# EUROPEAN PATENT SPECIFICATION

(11) **EP 3 643 277 B1**
(45) Date of publication and mention of the grant of the patent: **29.05.2024**
(21) Application number: 18821337.5
(22) Date of filing: 07.03.2018
(51) Int. Cl.: A61F 13/15

(54) **ABSORBENT ARTICLE PRODUCTION METHOD**
VERFAHREN ZUR HERSTELLUNG EINES SAUGFÄHIGEN ARTIKELS
PROCÉDÉ DE PRODUCTION D'UN ARTICLE ABSORBANT

(30) Priority: 20.06.2017 JP 2017120199
(43) Date of publication of application: 29.04.2020
(73) Proprietor: Unicharm Corporation, Shikokuchuo-shi, Ehime 799-0111 (JP)
(72) Inventor: HAGITA, Hiromi, Kanonji-shi Kagawa 769-1602 (JP); Tashiro Fumihiro, Kanonji-shi Kagawa 769-1602 (JP)
(74) Representative: Staeger & Sperling Partnerschaftsgesellschaft mbB
(86) International application number: PCT/JP2018/008844
(87) International publication number: WO 2018/235349

(56) References cited:
- EP-A1- 3 047 829
- JP-A- 2003 145 485
- JP-A- 2007 282 972
- JP-A- 2008 514 349
- JP-A- 2014 531 268
- JP-B1- 6 089 155
- US-A1- 2002 148 548
- US-A1- 2010 305 740

## Description

### [Technical Field]

The present invention relates to a method for manufacturing an absorbent article.

### [Background Art]

There is known a method for manufacturing an absorbent article while materials are transported along transporting routes respectively corresponding thereto. Examples of such absorbent articles include tape-type disposable diapers, pull-on disposable diapers, sanitary napkins, and the like.

When manufacturing absorbent articles in a manufacturing line, there are cases where a specification of the to-be-manufactured absorbent articles is changed based on a customer request or the like. For example, there are cases where if a customer orders (requests) an absorbent article whose absorbent body is a little thicker than that in a ready-made article (off-the-shelf article), the manufacturing line is switched from manufacturing the ready-made article (first-specification absorbent article) to manufacturing the ordered absorbent article (second-specification absorbent article), which has a minor change from the ready-made article.

When making such a switch, sometimes changes are made to various types of manufacturing conditions at different positions along a transporting route.

### [Citation List]

### [Patent Literature]

[PTL 1] WO2016/191438. Further prior art in this technical field is disclosed in documents US 2002/148548 A1 and JP 2008 514349 A.

### [Summary of Invention]

### [Technical Problem]

Such a switch has conventionally been made after stopping the manufacturing line (i.e., after pausing the transporting of materials). However, this method has the problem of a reduction in productivity. In view of this, it is conceivable to make the switch without stopping the manufacturing line (i.e., while the transporting of materials is maintained). However, in such a case, sometimes produced are absorbent articles that include both the first specification and the second specification (defective products).

The present invention was achieved in light of conventional problems such as that described above, and an aspect of the present invention is to reduce the number of defective absorbent articles.

### [Solution to Problem]

The present invention for achieving the above-described aspect is defined by a method according to claim 1 for manufacturing an absorbent article including:
manufacturing a first-specification absorbent article while transporting a material along a transporting route that corresponds to the material; and
switching from manufacturing of the first-specification absorbent article to manufacturing of a second-specification absorbent article by changing a plurality of types of manufacturing conditions at different positions in the transporting route while the transporting of the material is maintained,
   the plurality of types of manufacturing conditions including:
      a first manufacturing condition that is changed at a first position in the transporting route; and
      a second manufacturing condition that is changed at a second position that is downstream of the first position,
   the switching including setting timings for changing the manufacturing conditions so that a timing for changing the second manufacturing condition is later than a timing for changing the first manufacturing condition.

Features of the present invention other than the above will become clear by reading the description of the present specification with reference to the accompanying drawings.

### [Advantageous Effects of Invention]

According to the present invention, it is possible to reduce the number of defective absorbent articles.

### [Brief Description of the Drawings]

FIG. 1 is a schematic view of a manufacturing line for an absorbent article 1.
FIG. 2 is an illustrative schematic view for describing a comparative example.
FIG. 3 is a schematic view of a manufacturing line according to the first embodiment.
FIG. 4 is a flowchart according to the first embodiment.
FIG. 5 is a conceptual view of graphics that are to be printed on a first-specification absorbent article 1 and a second-specification absorbent article 1.
FIG. 6 is a schematic view of a manufacturing line according to the second embodiment.
FIG. 7 is a flowchart according to the second embodiment.
FIG. 8 is a schematic view of a manufacturing line according to the third embodiment.
FIG. 9 is a flowchart according to the third embodiment.

### [Description of Embodiments]

At least the following matters will become clear with the description of this specification and the attached drawings.

A method for manufacturing an absorbent article including:
manufacturing a first-specification absorbent article while transporting a material along a transporting route that corresponds to the material; and
switching from manufacturing of the first-specification absorbent article to manufacturing of a second-specification absorbent article by changing a plurality of types of manufacturing conditions at different positions in the transporting route while the transporting of the material is maintained,
   the plurality of types of manufacturing conditions including:
      a first manufacturing condition that is changed at a first position in the transporting route; and
      a second manufacturing condition that is changed at a second position that is downstream of the first position,
   the switching including setting timings for changing the manufacturing conditions so that a timing for changing the second manufacturing condition is later than a timing for changing the first manufacturing condition.

According to this method for manufacturing an absorbent article, it is possible to reduce the number of defective absorbent articles.

In such a method for manufacturing an absorbent article, it is desirable that
in the switching, the plurality of types of manufacturing conditions are changed in order from an upstream side in the transporting route.

According to this method for manufacturing an absorbent article, it is possible to more appropriately reduce the number of defective absorbent articles.

In such a method for manufacturing an absorbent article, it is desirable
that a thickness of an absorbent body is different between the first-specification absorbent article and the second-specification absorbent article, and
that depending on a magnitude of the difference in thickness, it is determined whether or not a pressing condition for pressing the absorbent body is to be changed in the switching as a manufacturing condition.

According to this method for manufacturing an absorbent article, rather than always changing the pressing condition whenever the specification of the absorbent article is changed, the pressing condition is not changed if the magnitude of the difference is very small for example. This makes it possible to avoid the production of defective products in such a case. Also, when it has been determined that the pressing condition is to be changed, the number of defective products can be reduced by delaying the timing for changing.

In such a method for manufacturing an absorbent article, it is desirable
that a thickness of an absorbent body is different between the first-specification absorbent article and the second-specification absorbent article, and
that depending on a magnitude of the difference in thickness, it is determined whether or not an embossing condition for forming embossing in the absorbent body is to be changed in the switching as a manufacturing condition.

According to this method for manufacturing an absorbent article, rather than always changing the embossing condition if the specification of the absorbent article is changed, the embossing condition is not changed if the magnitude of the difference is very small for example, thus making it possible to avoid the production of defective products in such a case. Also, when it has been determined that the embossing condition is to be changed, the number of defective products can be reduced by delaying the change timing.

In such a method for manufacturing an absorbent article, it is desirable
that a basis weight of an absorbent body is different between the first-specification absorbent article and the second-specification absorbent article, and
that depending on a magnitude of the difference in basis weight, it is determined whether or not a pressing condition for pressing the absorbent body is to be changed in the switching as a manufacturing condition.

According to this method for manufacturing an absorbent article, rather than always changing the pressing condition whenever the specification of the absorbent article is changed, the pressing condition is not changed if the magnitude of the difference is very small for example. This makes it possible to avoid the production of defective products in such a case. Also, when it has been determined that the pressing condition is to be changed, the number of defective products can be reduced by delaying the timing for changing.

In such a method for manufacturing an absorbent article, it is desirable
that a basis weight of an absorbent body is different between the first-specification absorbent article and the second-specification absorbent article, and
that depending on a magnitude of the difference in basis weight, it is determined whether or not an embossing condition for forming embossing in the absorbent body is to be changed in the switching as a manufacturing condition.

According to this method for manufacturing an absorbent article, rather than always changing the embossing condition whenever the specification of the absorbent article is changed, the embossing condition is not changed if the magnitude of the difference is very small for example. This makes it possible to avoid the production of defective products in such a case. Also, when it has been determined that the embossing condition is to be changed, the number of defective products can be reduced by delaying the timing for changing.

In such a method for manufacturing an absorbent article, it is desirable
that the absorbent article includes an elastic member,
that a stress of the elastic member is different between the first-specification absorbent article and the second-specification absorbent article, and
that depending on a magnitude of the difference in stress, it is determined whether or not an adhesive application condition for applying an adhesive to the elastic member is to be changed in the switching as a manufacturing condition.

According to this method for manufacturing an absorbent article, rather than always changing the adhesive application condition whenever the specification of the absorbent article is changed, the adhesive application condition is not changed if the magnitude of the difference is very small for example. This makes it possible to avoid the production of defective products in such a case. Also, when it has been determined that the adhesive application condition is to be changed, the number of defective products can be reduced by delaying the timing for changing.

In such a method for manufacturing an absorbent article, it is according to the invention that
while the absorbent article is manufactured,
a sheet-shaped material is fed and transported as the material, from a material coil on which the sheet-shaped material is wound,
at least one of a width, a thickness, and a basis weight of the sheet-shaped material is changed in the switching as a manufacturing condition, and
depending on a magnitude of change in the at least one of the width, the thickness, and the basis weight,
   it is determined whether or not a tension condition of the sheet-shaped material is to be changed in the switching as a manufacturing condition.

According to this method for manufacturing an absorbent article, rather than always changing the tension condition whenever at least one of the width, the thickness, and the basis weight of the sheet-shaped material is changed, the tension condition is not changed if the magnitude of change is very small for example. This makes it possible to avoid the production of defective products in such a case. Also, when it has been determined that the tension condition is to be changed, the number of defective products can be reduced by delaying the timing for changing.

In such a method for manufacturing an absorbent article, it is desirable
that while the absorbent article is manufactured, inspection of an absorbent body is executed by an inspection unit,
that the absorbent body includes pulp fibers,
that a basis weight of the pulp fibers is different between the first-specification absorbent article and the second-specification absorbent article, and
that depending on a magnitude of the difference in basis weight of the pulp fibers, it is determined whether or not an inspection condition of the inspection unit is to be changed in the switching as a manufacturing condition.

According to this method for manufacturing an absorbent article, rather than always changing the inspection condition whenever the specification of the absorbent article is changed, the inspection condition is not changed if the magnitude of the difference is very small for example. This makes it possible to avoid the production of defective products in such a case. Also, when it has been determined that the inspection condition is to be changed, the number of defective products can be reduced by delaying the timing for changing.

In such a method for manufacturing an absorbent article, it is disclosed
that a graphic is formed on the absorbent article, and
that in the switching, the graphic formation condition for forming the graphic is changed as a manufacturing condition such that a specification-related indication is changed with a common indication remaining unchanged.

According to this method for manufacturing an absorbent article, it is possible to minimize the change to the graphic formation condition while also appropriately informing the user that the specification was changed.

### Absorbent article manufacturing method according to embodiments

A method for manufacturing an absorbent article according to the present embodiment is carried out using an absorbent-article manufacturing device 10 (manufacturing line) for manufacturing an absorbent article 1. In other words, the method for manufacturing an absorbent article of the present embodiment is for manufacturing the absorbent article 1.

The absorbent article 1 can be exemplified by an open-type or pull-on disposable diaper, sanitary napkin, or the like. The absorbent article 1 may be any article as long as it absorbs excreted fluid from the wearer.

The absorbent article 1 includes: an absorbent body 2 that absorbs excreted fluid; a liquid-permeable top sheet that is arranged on the skin side of the absorbent body in the thickness direction; a liquid-impermeable back sheet that is arranged on the non-skin side of the absorbent body and prevents the leakage of excreted fluid to the non-skin side; and elastic strings 9, which are an example of elastic members. The back sheet includes a back film and a back nonwoven fabric sheet, which are overlaid on each other to form the back sheet.

The absorbent body 2 includes an absorbent core constituted by a molded liquid absorbent material. The liquid absorbent material can include pulp fibers and a superabsorbent polymer (SAP) for example, and these materials are used here. Examples of the material forming the top sheet and the back nonwoven fabric sheet include a nonwoven fabric sheet that contains thermoplastic resin fibers made of polyethylene, polypropylene, or the like. The back film can be formed using a thermoplastic resin film made of polyethylene or the like.

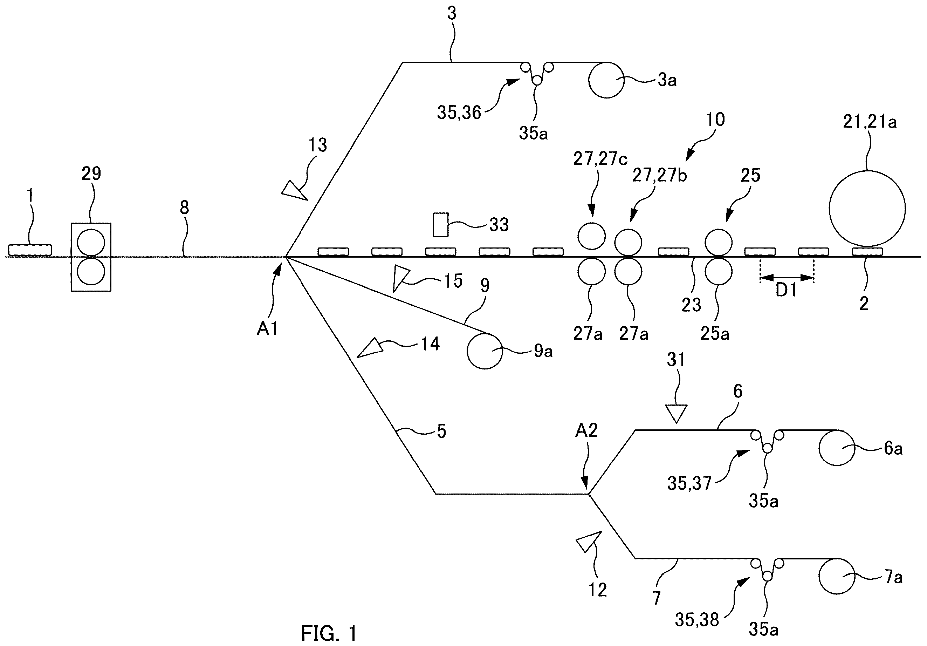

FIG. 1 is a schematic view of a manufacturing line for the absorbent article 1.

A top sheet material 3 is a sheet-shaped material for forming the top sheet, and the top sheet material 3 is brought into the manufacturing line in the form of a material coil 3a in which the top sheet material 3 has been wound in a coil-like manner. Specifically, the top sheet material 3 is fed from the material coil 3a, which is a coil of the top sheet material 3, and the fed top sheet material 3 is transported in a direction of transport (a direction in which the top sheet material 3 is continued) by a transporting mechanism (not shown). The top sheet material 3 then arrives at a merging position A1 where it merges with a later-described back sheet material 5.

Similarly, a back film material 6 and a back-nonwoven-fabric-sheet material 7, which are both sheet-shaped materials for forming the back sheet (back film and back nonwoven fabric sheet), are brought into the manufacturing line in the form of material coils 6a and 7a in which the back film material 6 and the back-nonwoven-fabric-sheet material 7 have been wound in a coil-like manner. Specifically, the back film material 6 and the back-nonwoven-fabric-sheet material 7 are fed from the material coils 6a and 7a of the back film material 6 and the back-nonwoven-fabric-sheet material 7, and the fed back film material 6 and back-nonwoven-fabric-sheet material 7 are transported in a direction of transport (a direction in which the top sheet material 3 the back film material 6 and the back-nonwoven-fabric-sheet material 7 are continued) by a transporting mechanism (not shown). The back film material 6 and the back-nonwoven-fabric-sheet material 7 then merge at a merging position A2, and are bonded together using an adhesive (a hot-melt adhesive in the present embodiment) to form the back sheet material 5.

Note that the hot-melt adhesive is applied to the back-nonwoven-fabric-sheet material 7 and not to the back film material 6, at a position before the merging position A2. Specifically, an adhesive application device (first application device 12) applies the hot-melt adhesive to the back-nonwoven-fabric-sheet material 7 on the upstream side in the direction of transport with respect to the merging position A2.

Also, in the present embodiment, a graphic is formed on the absorbent article 1, and this graphic is printed on the back film material 6 by an inkjet device 31. Specifically, the inkjet device 31 prints the graphic on the back film material 6 on the upstream side in the direction of transport with respect to the merging position A2.

The produced back sheet material 5 is transported in a direction of transport (a direction in which the back sheet material 5 is continued) by a transporting mechanism (not shown), and then arrives at the merging position A1 where it merges with the top sheet material 3.

Also, the absorbent body 2 (which is a material and is part of the product) is produced by shaping pulp fibers and a superabsorbent polymer (SAP) into a cutform shape. The absorbent body 2 is produced by a fiber-depositing drum device 21 that has a rotating drum 21a and a spraying duct (not shown). Recessed portions are formed in the outer circumferential surface of the rotating drum 21a at a product pitch D1 in the direction of rotation. The pulp fibers and the superabsorbent polymer (SAP) are sprayed toward the outer circumferential surface (recessed portions) by the spraying duct. The pulp fibers and the superabsorbent polymer (SAP) accumulate in the recessed portions, and thus the absorbent body 2 is formed in each of the recessed portions. The absorbent bodies 2 in the recessed portions are transferred to a belt conveyor 23, and then the absorbent bodies 2 are transported by the belt conveyor 23 in the direction of transport side-by-side at the product pitch D1.

The absorbent body 2 then arrives at an absorbent-body pressing device 25 and is pressed by the absorbent-body pressing device 25. The absorbent-body pressing device 25 includes two press rollers 25a that face each other, and the size of a gap (clearance) between the press rollers 25a is controlled. By passing through the gap (clearance), the absorbent body 2 is pressed (compressed).

After passing through the absorbent-body pressing device 25, the absorbent body 2 is further transported in the direction of transport and then arrives at an embossing device 27. Embossing is formed in the absorbent body 2 by the embossing device 27. This embossing makes it possible to control excreted fluid spreading and the like in the absorbent body 2. The embossing device 27 includes two embossing rollers 27a that face each other, and embossing is formed in the absorbent body 2 by the absorbent body 2 being pressed between the embossing rollers 27a. Note that although this will be described in detail later, in order to manufacture absorbent articles 1 that have different specifications (a first-specification absorbent article 1 and a second-specification absorbent article 1), the following two embossing devices 27 are prepared (for a first specification and a second specification) in the present embodiment: a first embossing device 27b for the first specification; and a second embossing device 27c for the second specification.

After passing through the embossing device 27, the absorbent body 2 is further transported in the direction of transport and then arrives at the merging position A1 where it merges with the top sheet material 3 and the back sheet material 5.

At the merging position A1, the absorbent body 2, the top sheet material 3, and the back sheet material 5 are bonded together with use of a hot-melt adhesive, and thus a base sheet 8 is produced.

Note that the hot-melt adhesive is applied to the top sheet material 3 and the back sheet material 5 at a position before the merging position A1. Specifically, an adhesive application device (second application device 13) applies the hot-melt adhesive to the top sheet material 3 on the upstream side in the direction of transport with respect to the merging position A1, and an adhesive application device (third application device 14) applies the hot-melt adhesive to the back sheet material 5 on the upstream side in the direction of transport with respect to the merging position A1.

Also, elastic strings 9 (which are materials and are part of the product) are also transported to the merging position A1, and the elastic strings 9 are also bonded together with the absorbent body 2, the top sheet material 3, and the back sheet material 5 (i.e., the elastic strings 9 are included in the base sheet 8). The elastic strings 9 are fed from a holding unit on which the elastic strings 9 have been wound in a coil-like manner (for convenience, the holding unit will be called an elastic-string coil 9a), and the fed elastic strings 9 are transported in a direction of transport (a direction in which the elastic strings 9 are continued) by a transporting mechanism (not shown). A hot-melt adhesive is applied to the elastic strings 9 at a position before the merging position A1. Specifically, an adhesive application device (fourth application device 15) applies the hot-melt adhesive to the elastic strings 9 on the upstream side in the direction of transport with respect to the merging position A1.

The produced base sheet 8 is further transported in the direction of transport and then arrives at an end cutter 29. The base sheet 8 is cut by the end cutter 29, thus manufacturing the absorbent article 1.

Note that in the present embodiment, the absorbent article 1 (absorbent body 2) is inspected by an inspection camera device 33, which is one example of an inspection unit. The inspection camera device 33 captures an image of the absorbent body 2 at a position in the transporting route that is downstream of the embossing device 27 and upstream of the merging position A1, and performs binarization image processing on the captured image. The inspection camera device 33 then determines whether or not a defect exists in the absorbent article 1 (absorbent body 2) by determining that black portions are normal portions where the liquid absorbent material is present, and that white portions are abnormal portions where the liquid absorbent material is not present.

Also, in the present embodiment, in order to control the tensions (tensile forces) of the sheet materials (top sheet material 3, back film material 6, and back-nonwoven-fabric-sheet material 7), a tension adjustment device 35 (so-called dancer unit) is provided for each of the sheet materials. Specifically, a tension adjustment device 35 (first tension adjustment device 36) for controlling the tension (tensile force) of the top sheet material 3 is provided downstream of the material coil 3a in the transporting route. Also, a tension adjustment device 35 (second tension adjustment device 37) for controlling the tension (tensile force) of the back film material 6 is provided downstream of the material coil 6a in the transporting route, and a tension adjustment device 35 (third tension adjustment device 38) for controlling the tension (tensile force) of the back-nonwoven-fabric-sheet material 7 is provided downstream of the material coil 7a in the transporting route. Such tensions (tensile forces) are respectively adjusted by changes in the positions of moving rolls 35a of the tension adjustment devices 35.

### Switching step according to present embodiment

There are cases where the specifications of the manufactured absorbent article 1 are changed based on a customer request or the like while the absorbent article 1 is being manufactured on the manufacturing line (absorbent-article manufacturing device 10). For example, there are cases where if a customer orders (requests) an absorbent article 1 that has an absorbent body 2 that is a little thicker than that in a ready-made article (off-the-shelf article), the manufacturing line (absorbent-article manufacturing device 10) is switched from manufacturing of the ready-made article (first-specification absorbent article 1) to manufacturing of the ordered article (second-specification absorbent article 1), which has a minor change from the ready-made article.

When making such a switching, sometimes changes are made to various types of manufacturing conditions at different positions along transporting routes. For example, a manufacturing condition (absorbent-body-material supply condition) of the fiber-depositing drum device 21 is changed in order to increase the amount of the liquid absorbent material (pulp fibers and superabsorbent polymer (SAP)), and furthermore a manufacturing condition (pressing condition) of the absorbent-body pressing device 25 is changed (i.e., the clearance is set larger).

Such a switching has conventionally been made after stopping the manufacturing line (i.e., after pausing the transporting of materials). However, this method has the problem of a reduction in productivity. In view of this, it is conceivable to make the switching without stopping the manufacturing line (i.e., while the transporting of materials is maintained). However, other problems such as the following can occur in such a case.

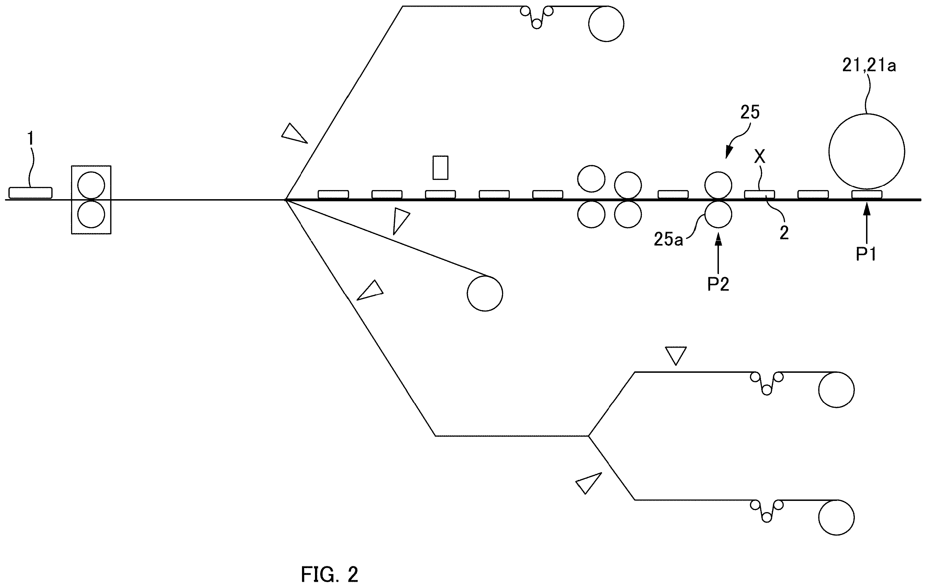

These problems will be described below with reference to FIG. 2. FIG. 2 is an illustrative schematic view for describing a comparative example. In the case where the previously mentioned order (request) is received while carrying out a manufacturing step for manufacturing the first-specification absorbent article 1, multiple types of manufacturing conditions at different positions on transporting routes are changed while materials continue to be transported. Specifically, a manufacturing condition of the fiber-depositing drum device 21 (absorbent-body-material supply condition) is changed at a position P1, and a manufacturing condition of the absorbent-body pressing device 25 (pressing condition) is changed at a position P2.

However, when such changes are made, the absorbent body 2 denoted by reference sign X has already be formed with the pre-change absorbent-body-material supply condition, and even though the amount of liquid absorbent material corresponds to the first-specification absorbent article 1, that absorbent body 2 is pressed with the changed pressing condition, that is to say the pressing condition that corresponds to the second-specification absorbent article 1. Accordingly, the absorbent article 1 will include both the first specification and the second specification (such an absorbent article 1 is a defective product).

Concerning the multiple types of manufacturing conditions which include a first manufacturing condition (i.e., the absorbent-body-material supply condition) and a second manufacturing condition (i.e., the pressing condition), it is assumed that the first manufacturing condition is changed at a first position (i.e., the position P1) in the transporting route and a second manufacturing condition that is changed at a second position (i.e., the position P2) located downstream with respect to the first position (position P1). In view of the foregoing paragraphs, in the present embodiment, the timings for changing the manufacturing conditions are set so that the timing for changing the second manufacturing condition is later than the timing for changing the first manufacturing condition. According to this configuration, to the absorbent body 2 denoted by the reference sign X, it is possible to apply the absorbent-body-material supply condition and the pressing condition that were set before the change. This therefore makes it possible to reduce the number of absorbent articles 1 that have both the first specification and the second specification, that is to say the number of defective products.

Whereas the above description has been given by way of a simple example of changing only two types of manufacturing conditions in order to facilitate understanding the gist of the present invention, the following describes the present invention by way of three embodiments (called first to third embodiments) pertaining to more realistic examples.

### First Embodiment

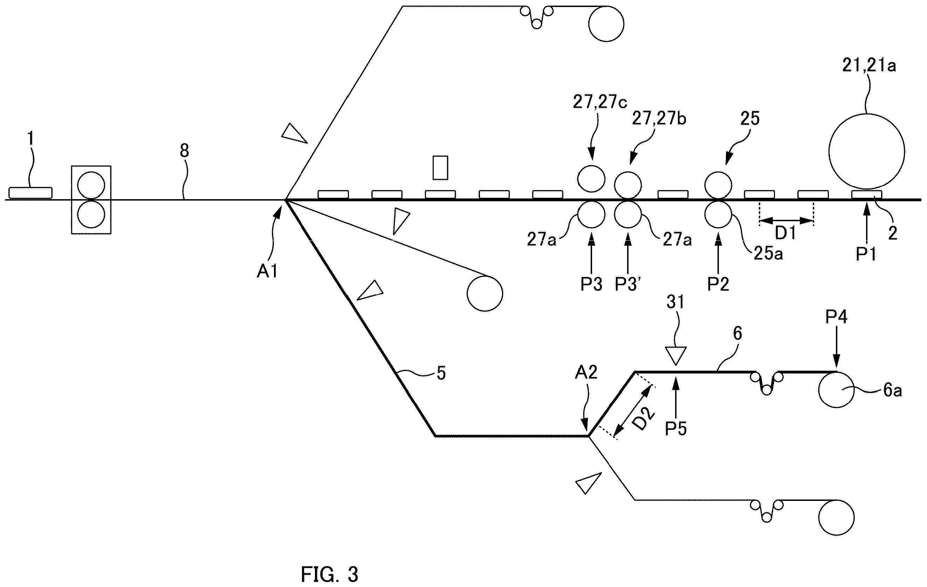

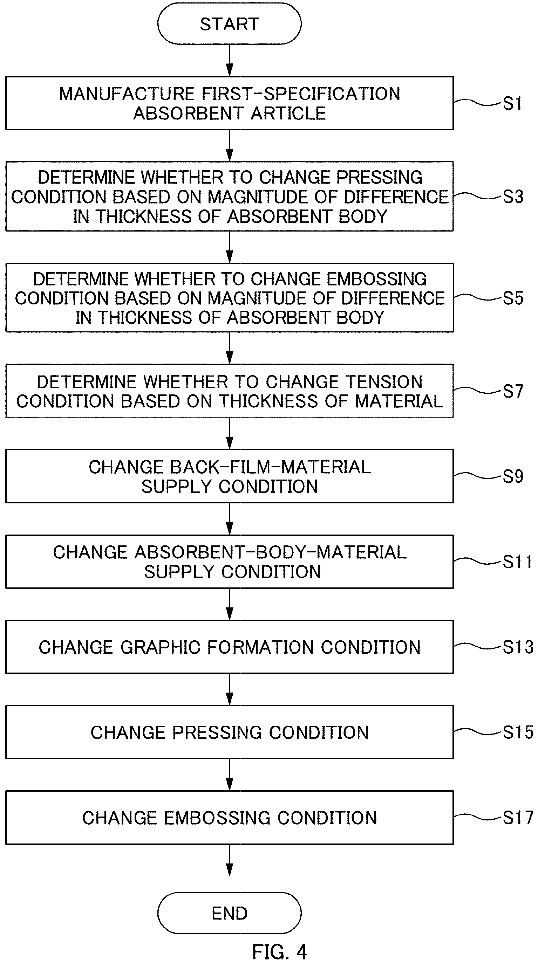

FIG. 3 is a schematic view of a manufacturing line according to the first embodiment. FIG. 4 is a flowchart according to the first embodiment.

First, assume that while a manufacturing step for manufacturing a ready-made article (first-specification absorbent article 1) is being carried out (step S1), an order (request) is received for an absorbent article 1 that has an absorbent body 2 and a back film that are a little thicker than that in the ready-made article. This order (request) envisions a product that can handle a little more excreted fluid than normal.

In such a case, the manufacturing line (absorbent-article manufacturing device 10) is switched from manufacturing of the ready-made article (first-specification absorbent article 1) to manufacturing of the ordered article (second-specification absorbent article 1), which has a minor change from the ready-made article. But before this switching is executed, a determination is made regarding whether or not several manufacturing conditions are to be changed.

Specifically, in the present embodiment, a manufacturing condition of the fiber-depositing drum device 21 (absorbent-body-material supply condition) is changed to increase the amount of liquid absorbent material (pulp fibers and superabsorbent polymer (SAP)). A manufacturing condition of the absorbent-body pressing device 25 (pressing condition) and a manufacturing condition of the embossing device 27 (embossing condition) may be changed or not changed depending on the magnitude of the difference in thickness of the absorbent body 2 (i.e., how much the thickness of the absorbent body 2 changes when changing from the first specification to the second specification). This is because if the difference in thickness of the absorbent body 2 is very small (e.g., within a 10% decrease or 10% increase), there is no need to change such manufacturing conditions (pressing condition and embossing condition). Here, assume that based on the determination made according to the magnitude of the difference in thickness of the absorbent body 2 (step S3 and step S5), it is determined that both the pressing condition and the embossing condition are to be changed.

Also, a sheet-shaped-material supply condition (here, a back-film-material supply condition) is changed in order to supply a thicker back film material 6. At this time, the tension condition (specifically, the tension value) of the back film material 6 may or may not be changed depending on the magnitude of change in the thickness of the back film material 6. This is because if the magnitude of change in the thickness of the back film material 6 is very small (e.g., within a 10% decrease or 10% increase), there is no need to change the tension condition, but if the magnitude of change is large, the tension condition needs to be changed. Here, assume that based on the determination made according to the magnitude of change in the thickness of the back film material 6 (step S7), it is determined that the tension condition is not to be changed.

Also, in the present embodiment, when the specification of the absorbent article 1 is changed from the first specification to the second specification, the graphic is changed in correspondence with the change in specification. Accordingly, a manufacturing condition of the inkjet device 31 (graphic formation condition) is changed in order to change the graphic.

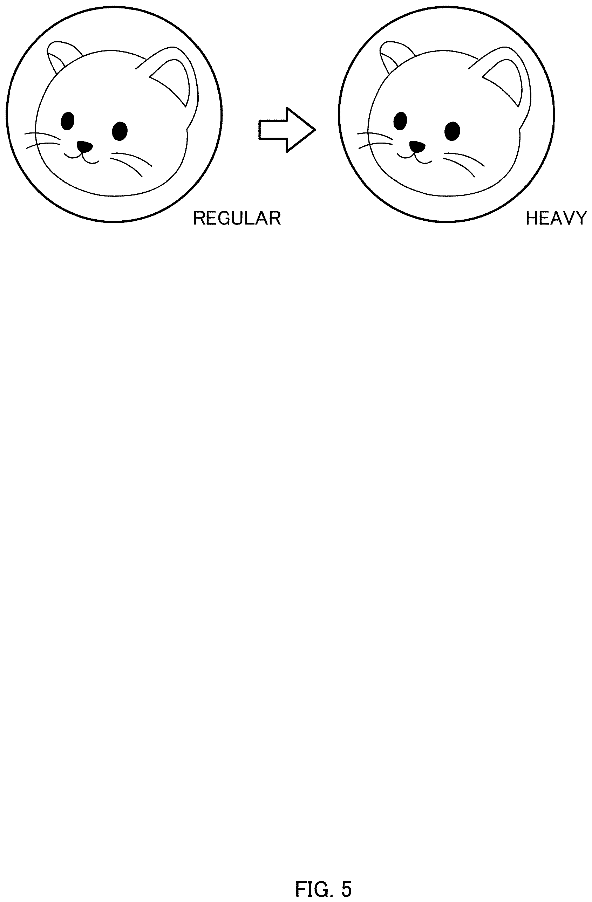

FIG. 5 is a conceptual view of graphics that are to be printed on a first-specification absorbent article 1 and a second-specification absorbent article 1. The left side shows the graphic that corresponds to the first specification, and the right side shows the graphic that corresponds to the second specification. In this way, in the present embodiment, the common indication (a character depiction in the present embodiment) remains unchanged, but the specification-related indication is changed (from "regular" to "heavy" in the present embodiment). The graphic formation condition is changed such that this continuity and change are realized.

As described above, in the present embodiment, when the manufacturing line (absorbent-article manufacturing device 10) is switched from manufacturing of the ready-made article (first-specification absorbent article 1) to manufacturing of the ordered article (second-specification absorbent article 1), the absorbent-body-material supply condition, the pressing condition, the embossing condition, the back-film-material supply condition, and the graphic formation condition are changed.

For example, the absorbent-body-material supply condition is changed so as to increase the amount of liquid absorbent material (pulp fibers and superabsorbent polymer (SAP)).

Also, the pressing condition is changed so as to increase the clearance.

Also, the embossing-pattern formation condition is changed such that the embossing pattern corresponds to the thicker absorbent body 2. Specifically, the embossing pattern is changed by switching the embossing device 27 from the first embossing device 27b for the specification to the second embossing device 27c for the second specification. Note that because the embossing device is switched to the second embossing device 27c, the embossing condition is changed at a position P3, not at a position P3' in FIG. 3.

Also, the back-film-material supply condition is changed such that the back film material 6 is a thicker material. Specifically, preparing a material coil 6a on which a back film material 6 corresponding to the second specification (a thicker back film material 6) is wound, the corresponding-to-second-specification back film material 6 is joined to the currently transported corresponding-to-first-specification back film material 6 using a known method (this material joining can be performed without stopping the transporting of the back film material 6). Note that the position at which the back-film-material supply condition is changed (a position P4 in FIG. 3) is the position where the material is joined.

Also, the graphic formation condition is changed such that the graphic shown on the right side in FIG. 5 is formed.

When such changes are made, concerning the multiple types of manufacturing conditions which includes a first manufacturing condition and a second manufacturing condition, in the case where the first manufacturing condition is changed at a first position in the transporting route and the second manufacturing condition is changed at a second position located downstream with respect to the first position, the timings for changing the manufacturing conditions are set so that the timing for changing the second manufacturing condition is later than the timing for changing the first manufacturing condition. Furthermore, in the present embodiment, among the five manufacturing conditions, the delay of the timing for changing is increased the further downstream the position is. In other words, the five manufacturing conditions are changed in order from the upstream side in the transporting route.

The following describes the definition of "upstream (downstream) in the transporting route". In other words, the following describes a method for specifying which of multiple positions is upstream (downstream) of the others. First, where the "transporting route" is depends on the position where the manufacturing condition is changed. Specifically, in the previously-described simple example, only the positions P1 and P2 correspond to the aforementioned positions. Accordingly, the "transporting route" is the absorbent-body-2-moving-route (belt conveyor 23) on which both the positions P1 and P2 are located (see the bold line in FIG. 2). In this case, the "transporting route" is a single line and does not have a merge (branch), and therefore the position that is forward in the direction of transport is on the upstream side, and the position that is backward in the direction of transport is on the downstream side.

Also, in the first embodiment, as shown in FIG. 3, the absorbent-body-material supply condition is changed at the position P1, the pressing condition is changed at the position P2, the embossing condition is changed at the position P3, the back-film-material supply condition is changed at the position P4, and the graphic formation condition is changed at a position P5. For this reason, the "transporting route" is a combination of the moving route of the absorbent body 2 and the moving route of the back film material 6 (and the back sheet material 5) (see the bold lines in FIG. 3). In other words, unlike the previously-described simple example, the "transporting route" of the first embodiment is a line that has a merge (branch).

In this case, the most downstream merging position (a merging position A1 in this example because there is only one merging position) serves as the reference when determining whether a position is "upstream (downstream)". Furthermore, this determination is made based on a pitch count, which corresponds to the number of product pitches (using product pitch as a unit).

This will be described specifically below. Let L1 be the distance (length of the moving route) from the merging position A1 (reference) to the position P1, and let D1 (see FIG. 3) be the product pitch in the moving route of the absorbent body 2. Let N1 be the pitch count from the merging position A1 to the position P1 (N1 = L1/D1). Using a similar method, it is possible to obtain a pitch count N2 from the merging position A1 to the position P2, a pitch count N3 from the merging position A1 to the position P3, a pitch count N4 from the merging position A1 to the position P4, and a pitch count N5 from the merging position A1 to the position P5. When comparing two positions, the position having the larger pitch count is determined to be on the upstream side. Here, N3 < N2 < N5 < N1 < N4, and therefore the position P3 is the most downstream, and the position P2, the position P5, the position P1, and the position P4 are increasingly upstream in this order. Note that the product pitch is normally different between the moving route of the absorbent body 2 and the moving route of the back film material 6 (see the lengths D1 and D2 in FIG. 3), and therefore sometimes the "upstream (downstream)" determination is mistakenly made simply using distance (a determination made based on the pitch count and a determination made based on distance will not necessarily match each other).

Note that, needless to say, this determination made based on the pitch count is also applicable to the previously-described example in which the "transporting route" is a single line.

In the present embodiment, the five manufacturing conditions are changed in order from the upstream side in the transporting route. Specifically, first, the back-film-material supply condition is changed (step S9). Thereafter, after a time delay, the absorbent-body-material supply condition is changed (step S11). Thereafter, after a time delay, the graphic formation condition is changed (step S13). Thereafter, after a time delay, the pressing condition is changed (step S15). Thereafter, after a time delay, the embossing condition is changed (step S17). Note that when determining the length of the time delay, from the viewpoint of minimizing the number of defective products, it is desirable that the time delay is a value obtained by dividing the difference between the pitch counts of the two (forward and backward) manufacturing conditions by the production speed (the number of absorbent articles that are manufactured per second). For example, letting the production speed be S (products/seconds), it is desirable that the absorbent-body-material supply condition is changed at a timing when (N4 - N1)/S (seconds) has passed since the back-film-material supply condition has been changed.

As described above, the method for manufacturing an absorbent article of the present embodiment includes: the manufacturing step of transporting materials (absorbent body 2, top sheet material 3, back film material 6, back-nonwoven-fabric-sheet material 7, elastic strings 9, etc.) along material-corresponding transporting routes while manufacturing the first-specification absorbent article 1; and the switching step of switching from manufacturing of the first-specification absorbent article 1 to manufacturing of the second-specification absorbent article 1 by changing multiple types of manufacturing conditions at different positions on the corresponding transporting routes while the transporting of the materials is maintained. In the switching step, concerning the multiple types of manufacturing conditions which includes a first manufacturing condition and a second manufacturing condition, in the case where the first manufacturing condition is changed at a first position in the transporting route and the second manufacturing condition that is changed at a second position located downstream with respect to the first position, the timings for changing the manufacturing conditions are set so that the timing for changing the second manufacturing condition is later than the timing for changing the first manufacturing condition.

For this reason, as previously described, it is possible to reduce the number of absorbent articles 1 that have both the first specification and the second specification, that is to say the number of defective products.

Also, in the switching step of the present embodiment, the multiple types of manufacturing conditions are changed in order from the upstream side in the transporting route. In other words, all of the manufacturing conditions are reliably subjected to a process in which the timing for changing the second manufacturing condition at the second position located downstream with respect to the first position is later than the timing for changing the first manufacturing condition at the first position. For this reason, it is possible to more appropriately reduce the number of defective products.

Also, in the present embodiment, the thickness of the absorbent body 2 is different between the first-specification absorbent article 1 and the second-specification absorbent article 1. And as shown in step S3, depending on the magnitude of the difference in thickness, it is determined whether or not the pressing condition for pressing the absorbent body 2 is to be changed in the switching step as the manufacturing condition.

For this reason, rather than always changing the pressing condition whenever the specification of the absorbent article 1 is changed, the pressing condition is not changed if the magnitude of the difference is very small for example. This makes it possible to avoid the production of defective products in such a case. Also, when it has been determined that the pressing condition is to be changed, the number of defective products can be reduced by delaying the timing for changing as previously described.

Also, in the present embodiment, the thickness of the absorbent body 2 is different between the first-specification absorbent article 1 and the second-specification absorbent article 1. And as shown in step S5, depending on the magnitude of the difference in thickness, it is determined whether or not the embossing condition for forming embossing in the absorbent body 2 is to be changed in the switching step as the manufacturing condition.

For this reason, rather than always changing the embossing condition whenever the specification of the absorbent article 1 is changed, the embossing condition is not changed if the magnitude of the difference is very small for example. This makes it possible to avoid the production of defective products in such a case. Also, when it has been determined that the embossing condition is to be changed, the number of defective products can be reduced by delaying the timing for changing as previously described.

Also, in the present embodiment, the absorbent article 1 is manufactured as follow: the back film material 6 is fed and transported as a material, from the material coil 6a on which the back film material 6 is wound; at least one of the width, the thickness, and the basis weight of the back film material 6 (although the thickness is used in the present embodiment, this can also be similarly applied to cases of the width, or basis weight) is changed in the switching step as the manufacturing condition; and depending on the magnitude of change in the at least one of the width, the thickness, and the basis weight, it is determined whether or not the tension condition of the back film material 6 is to be changed in the switching step as the manufacturing condition.

For this reason, rather than always changing the tension condition whenever at least one of the width, the thickness, and the basis weight of the back film material 6 is changed, the tension condition is not changed if the magnitude of change is very small for example. This makes it possible to avoid the production of defective products in such a case. Also, when it has been determined that the tension condition is to be changed, the number of defective products can be reduced by delaying the timing for changing as previously described.

Note that although the example where the back film material 6 is the material is described in the present embodiment, there is no limitation to this, and the present invention is also applicable to another sheet-shaped material. For example, the present invention is also applicable to the top sheet material 3 and the back-nonwoven-fabric-sheet material 7, and in such a case, the determination of whether or not to change the tension condition is made based on the corresponding sheet-shaped material.

Also, in the absorbent article 1 of the present embodiment, a graphic is formed thereon, and in the switching step, the graphic formation condition is changed as the manufacturing condition such that the specification-related indication is changed with the common indication remaining unchanged.

For this reason, it is possible to minimize the change to the graphic formation condition while also appropriately informing the user that the specification was changed (a minor change was made to the specification).

### Second Embodiment

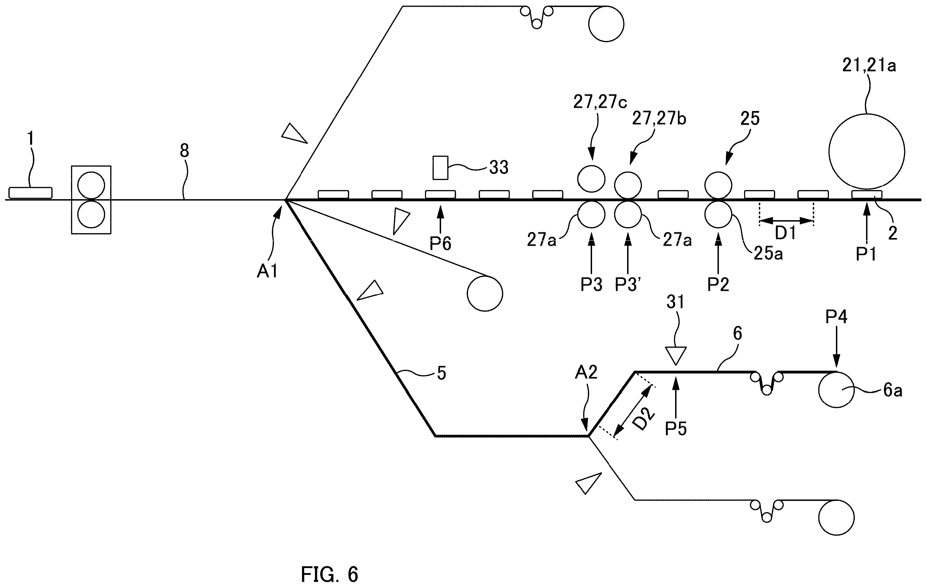

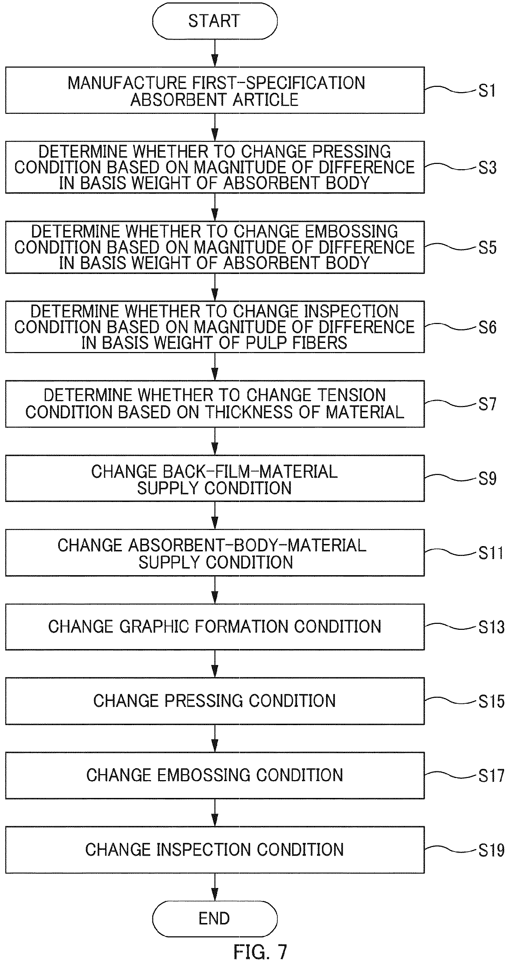

The following describes the second embodiment with reference to FIGS. 6 and 7. FIG. 6 is a schematic view of a manufacturing line according to the second embodiment. FIG. 7 is a flowchart according to the second embodiment.

In the first embodiment, an example is described in which the thickness of the absorbent body 2 is different between the first-specification absorbent article 1 and the second-specification absorbent article 1. But in the second embodiment, the basis weight of the absorbent body 2 is different instead of the thickness. Also, along with the difference in the basis weight of the absorbent body 2, the basis weight of the pulp fibers in the absorbent body 2 is also different. Therefore an inspection condition of the inspection camera device 33 (specifically, the threshold value in the binarization image processing) is changed such that an abnormality does not occur in the inspection.

First, assume that while a manufacturing step for manufacturing a ready-made article (first-specification absorbent article 1) is being carried out (step S1), an order (request) is received for an absorbent article 1 that has an absorbent body 2 with a little higher basis weight and a little thicker back film than in the ready-made article. This order (request) envisions a product that can handle a little more excreted fluid than normal.

In such a case, the manufacturing line (absorbent-article manufacturing device 10) is switched from manufacturing of the ready-made article (first-specification absorbent article 1) to manufacturing of the ordered article (second-specification absorbent article 1), which has a minor change from the ready-made article. But before this switching is executed, a determination is made regarding whether or not several manufacturing conditions are to be changed.

Specifically, in the present embodiment, a manufacturing condition of the fiber-depositing drum device 21 (absorbent-body-material supply condition) is changed to increase the amount of liquid absorbent material (pulp fibers and superabsorbent polymer (SAP)). A manufacturing condition of the absorbent-body pressing device 25 (pressing condition) and a manufacturing condition of the embossing device 27 (embossing condition) may be changed or not changed depending on the magnitude of the difference in basis weight of the absorbent body 2 (i.e., how much the basis weight of the absorbent body 2 changes when changing from the first specification to the second specification). This is because if the difference in basis weight of the absorbent body 2 is very small (e.g., within a 10% decrease or 10% increase), there is no need to change such manufacturing conditions (pressing condition and embossing condition). Here, assume that based on the determination made according to the magnitude of the difference in basis weight of the absorbent body 2 (step S3 and step S5), it is determined that both the pressing condition and the embossing condition are to be changed.

Also, an inspection condition of the inspection camera device 33 (specifically, the threshold value in the binarization image processing) may or may not be changed depending on the magnitude of the difference in basis weight of the pulp fibers. This is because if the magnitude of the difference in basis weight of the pulp fibers is very small (e.g., within a 10% decrease or 10% increase), it is possible to continue accurate inspection without changing the inspection condition, whereas if the magnitude of the difference is large, an inspection abnormality (misdiagnosis) will occur if the threshold value is not changed. Here, assume that based on the determination made according to the magnitude of the difference in basis weight of the pulp fibers (step S6), it is determined that the threshold value is to be changed.

Also, in step S7 and step S9, similarly to step S7 and step S9 of the first embodiment, the back-film-material supply condition and the graphic formation condition are changed, and the tension condition is not changed.

As described above, in the present embodiment, when the manufacturing line (absorbent-article manufacturing device 10) is switched from manufacturing of the ready-made article (first-specification absorbent article 1) to manufacturing of the ordered article (second-specification absorbent article 1), the absorbent-body-material supply condition, the pressing condition, the embossing condition, the back-film-material supply condition, the graphic formation condition, and the inspection condition are changed.

For example, the absorbent-body-material supply condition is changed at the position P1 so as to increase the amount of liquid absorbent material (pulp fibers and superabsorbent polymer (SAP)).

Also, the pressing condition is changed at the position P2 so as to increase the clearance.

Also, the embossing-pattern formation condition is changed at the position P3 such that the embossing pattern corresponds to the absorbent body 2 having a higher basis weight. Specifically, the embossing pattern is changed by switching the embossing device 27 from the first embossing device 27b for the specification to the second embossing device 27c for the second specification.

Also, the back-film-material supply condition is changed at the position P4 such that the back film material 6 is a thicker material.

Also, the graphic formation condition is changed at the position P5 such that the graphic shown on the right side in FIG. 5 is formed.

Also, the inspection condition (the threshold value in the binarization image processing) is changed at the position P6 such that even after the basis weight of the pulp fibers has changed, it is possible to correctly determine after binarization that a black portion is a normal portion and correctly determine that a white portion is an abnormal portion.

When such changes are made, concerning the multiple types of manufacturing conditions which includes a first manufacturing condition and a second manufacturing condition, in the case where the first manufacturing condition is changed at a first position in the transporting route and the second manufacturing condition is changed at a second position located downstream with respect to the first position, the timings for changing the manufacturing conditions are set so that the timing for changing the second manufacturing condition is later than the timing for changing the first manufacturing condition. Furthermore, in the present embodiment, among the six manufacturing conditions, the delay of the timing for changing is increased the further downstream the position is. In other words, the six manufacturing conditions are changed in order from the upstream side in the transporting route.

Specifically, the position P6 is the most downstream, and the position P3, the position P2, the position P5, the position P1, and the position P4 are increasingly upstream in this order, and therefore the back-film-material supply condition is changed first (step S9). Thereafter, after a time delay, the absorbent-body-material supply condition is changed (step S11). Thereafter, after a time delay, the graphic formation condition is changed (step S13). Thereafter, after a time delay, the pressing condition is changed (step S15). Thereafter, after a time delay, the embossing condition is changed (step S17). Thereafter, after a time delay, the inspection condition is changed (step S19).

As described above, in the second embodiment as well, similarly to the first embodiment, in the switching step, concerning the multiple types of manufacturing conditions which includes a first manufacturing condition and a second manufacturing condition, in the case where the first manufacturing condition is changed at a first position in the transporting route and the second manufacturing condition that is changed at a second position located downstream with respect to the first position, the timings for changing the manufacturing conditions are set so that the timing for changing the second manufacturing condition is later than the timing for changing the first manufacturing condition.

This therefore makes it possible to reduce the number of absorbent articles 1 that have both the first specification and the second specification, that is to say the number of defective products.

Also, the multiple types of manufacturing conditions are changed in order from the upstream side in the transporting route, thus making it possible to more appropriately reduce the number of defective products.

Also, in the present embodiment, the basis weight of the absorbent body 2 is different between the first-specification absorbent article 1 and the second-specification absorbent article 1. And as shown in step S3, depending on the magnitude of the difference in basis weight, it is determined whether or not the pressing condition for pressing the absorbent body 2 is to be changed in the switching step as the manufacturing condition.

For this reason, rather than always changing the pressing condition whenever the specification of the absorbent article 1 is changed, the pressing condition is not changed if the magnitude of the difference is very small for example. This makes it possible to avoid the production of defective products in such a case. Also, when it has been determined that the pressing condition is to be changed, the number of defective products can be reduced by delaying the timing for changing as previously described.

Also, in the present embodiment, the basis weight of the absorbent body 2 is different between the first-specification absorbent article 1 and the second-specification absorbent article 1. And as shown in step S5, depending on the magnitude of the difference in basis weight, it is determined whether or not the embossing condition for forming embossing in the absorbent body 2 is to be changed in the switching step as the manufacturing condition.

For this reason, rather than always changing the embossing condition whenever the specification of the absorbent article 1 is changed, the embossing condition is not changed if the magnitude of the difference is very small for example. This makes it possible to avoid the production of defective products in such a case. Also, when it has been determined that the embossing condition is to be changed, the number of defective products can be reduced by delaying the timing for changing as previously described.

Also, in the present embodiment, the absorbent article 1 is manufactured as follow: inspection of the absorbent body 2 is executed by the inspection camera device 33; the absorbent body 2 includes pulp fibers; the basis weight of the pulp fibers is different between the first-specification absorbent article 1 and the second-specification absorbent article 1; and depending on the magnitude of the difference in basis weight of the pulp fibers, it is determined whether or not the inspection condition of the inspection camera device 33 is to be changed in the switching step as the manufacturing condition.

For this reason, rather than always changing the inspection condition whenever the specification of the absorbent article 1 is changed, the inspection condition is not changed if the magnitude of the difference is very small for example. This makes it possible to avoid the production of defective products in such a case. Also, when it has been determined that the inspection condition is to be changed, the number of defective products can be reduced by delaying the timing for changing as previously described.

### Third Embodiment

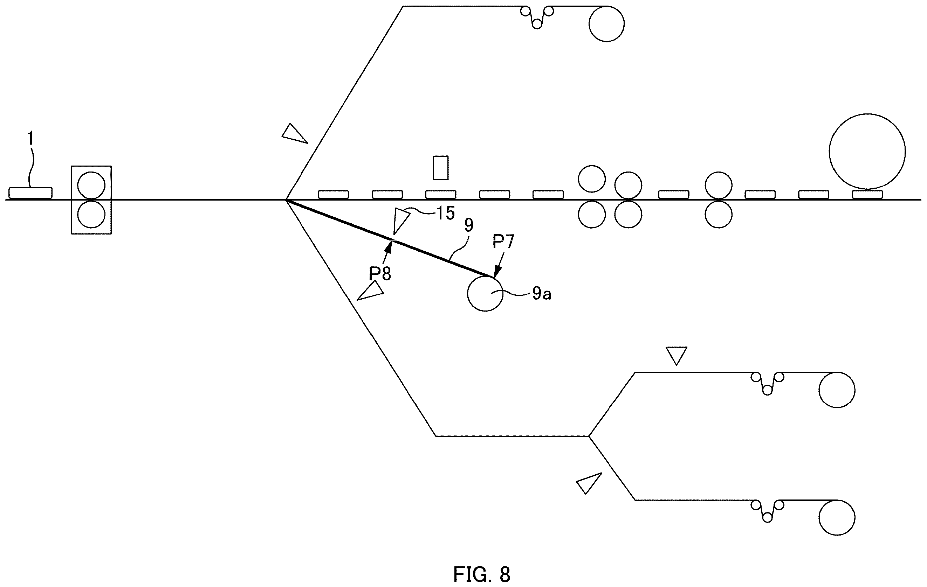

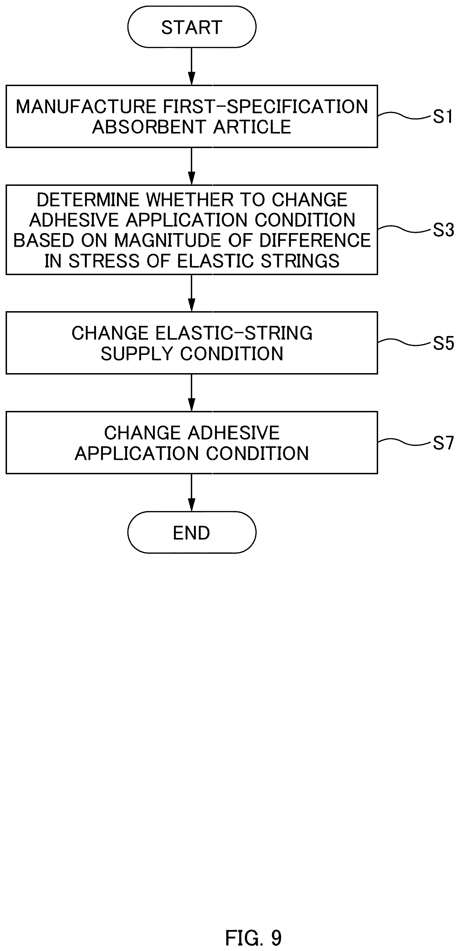

The following describes the third embodiment with reference to FIGS. 8 and 9. FIG. 8 is a schematic view of a manufacturing line according to the third embodiment. FIG. 9 is a flowchart according to the third embodiment.

Whereas cases of changing a specification related to the absorbent body 2 are described in the first and second embodiments, in the third embodiment, the stress of the elastic strings 9 is different between the first-specification absorbent article 1 and the second-specification absorbent article 1. Here, the stress of the elastic strings 9 is the contractive force per elastic string 9. In the present embodiment, an example is described in which the stress of the elastic strings 9 is changed by replacing the elastic strings 9 with elastic strings that have a higher stress, but as another example, the stretch factor of the elastic strings 9 may be changed.

First, assume that while a manufacturing step for manufacturing a ready-made article (first-specification absorbent article 1) is being carried out (step S1), an order (request) is received for an absorbent article 1 in which the stress of the elastic strings 9 is higher than that in the ready-made article. This order (request) envisions a product whose position is less likely to shift (less likely to fall) than normal when it is put on.

In such a case, the manufacturing line (absorbent-article manufacturing device 10) is switched from manufacturing of the ready-made article (first-specification absorbent article 1) to manufacturing of the ordered article (second-specification absorbent article 1), which has a minor change from the ready-made article. But before this switch is executed, a determination is made regarding whether or not an adhesive application condition for applying an adhesive to the elastic strings 9 is to be changed.

Specifically, in the present embodiment, the elastic-string supply condition is changed so as to supply elastic strings 9 that have a higher stress. Concerning a manufacturing condition of the fourth application device 15 (adhesive application condition; specifically a hot-melt-adhesive application amount), the adhesive application condition is either changed or not changed depending on the magnitude of the difference in stress (i.e., depending on how much the stress changes when changing from the first specification to the second specification). This is because if the difference in stress is very small (e.g., within a 10% decrease or 10% increase), there is no need to change the adhesive application condition (application amount). Here, assume that based on the determination made according to the magnitude of the difference in stress (step S3), it is determined that the adhesive application condition (application amount) is to be changed.

Accordingly, in the present embodiment, when the manufacturing line (absorbent-article manufacturing device 10) is switched from manufacturing of the ready-made article (first-specification absorbent article 1) to manufacturing of the ordered article (second-specification absorbent article 1), the elastic-string supply condition and the adhesive application condition are changed.

For example, the elastic-string supply condition is changed at a position P7 such that the elastic strings 9 have a higher stress.

Also, the adhesive application condition is changed at a position P8 such that the application amount is increased.

When such changes are made, concerning the multiple types of manufacturing conditions which includes a first manufacturing condition and a second manufacturing condition, in the case where the first manufacturing condition is changed at a first position in the transporting route and the second manufacturing condition is changed at a second position located downstream with respect to the first position, the timings for changing the manufacturing conditions are set so that the timing for changing the second manufacturing condition is later than the timing for changing the first manufacturing condition. In other words, first, the elastic-string supply condition is changed at the position P7 (step S5). Thereafter, after a time delay, the adhesive application condition is changed at the position P8 (step S7).

As described above, in the third embodiment as well, similarly to the first embodiment and the second embodiment, in the switching step, concerning the multiple types of manufacturing conditions which includes a first manufacturing condition and a second manufacturing condition, in the case where the first manufacturing condition is changed at a first position in the transporting route and the second manufacturing condition that is changed at a second position located downstream with respect to the first position, the timings for changing the manufacturing conditions are set so that the timing for changing the second manufacturing condition is later than the timing for changing the first manufacturing condition.

This therefore makes it possible to reduce the number of absorbent articles 1 that have both the first specification and the second specification, that is to say the number of defective products.

Also, in the present embodiment, the absorbent article 1 includes the elastic strings 9, the stress of the elastic strings 9 is different between the first-specification absorbent article 1 and the second-specification absorbent article 1, and depending on the magnitude of the difference in stress, it is determined whether or not the adhesive application condition for applying the adhesive to the elastic strings 9 is to be changed in the switching step as the manufacturing condition.

For this reason, rather than always changing the adhesive application condition whenever the specification of the absorbent article 1 is changed, the adhesive application condition is not changed if the magnitude of the difference is very small for example. This makes it possible to avoid the production of defective products in such a case. Also, when it has been determined that the adhesive application condition is to be changed, the number of defective products can be reduced by delaying the timing for changing as previously described.

### Other embodiments

Although the embodiments of the present disclosure have been described hereinabove, the above embodiments of the present disclosure are simply to facilitate understanding of the present disclosure and are not in any way to be construed as limiting the present disclosure. The present disclosure may variously be changed or altered without departing from its gist and encompass equivalents thereof.

In the above embodiments, examples of the manufacturing conditions include the sheet-shaped-material supply condition, the absorbent-body-material supply condition, the elastic-string supply condition, the pressing condition, the embossing condition, the inspection condition, the adhesive application condition, the graphic formation condition, and the tension condition, but there is no limitation to this. Another example that can be given is a modifier-agent application condition for applying a modifier agent. Examples of the modifier agent include a liquid or the like that includes a component for improving the dispersion and absorption of excreted fluid, a component for suppressing irritation of skin that comes into contact with the absorbent article, or a component for suppressing the smell of excreted fluid, for example.

Also, although the back-film-material supply condition is given as an example of the sheet-shaped-material supply condition, there is no limitation to this. Other examples include a top-sheet-material supply condition and a back-nonwoven-fabric-sheet material supply condition.

Also, although an example of changing the amount of pulp fibers and superabsorbent polymer (SAP) is given as an example of changing the absorbent-body-material supply condition in the above embodiments, there is no limitation to this. For example, the type of pulp fibers and superabsorbent polymer (SAP) may be changed.

Also, although an example of changing the size of the gap (clearance) is given as an example of changing the pressing condition, there is no limitation to this. For example, the following example is possible: there is no gap between the two press rollers 25a of the absorbent-body pressing device 25, and the extent of pressing is controlled by the amount of contact pressure between the two press rollers 25a, and the amount of contact pressure is changed. Also, the following another example is possible: the press rollers 25a are heated rollers, and the set temperature of the heated rollers is changed.

Also, although an example of changing the embossing pattern is given as an example of changing the embossing condition, there is no limitation to this. For example, the embossing pressure or positions may be changed.

Also, although the elastic strings 9 are given as an example of the elastic members in the above embodiments, there is no limitation to this. For example, sheet-shaped members that have elasticity may be used.

Also, in the above embodiments, based on the magnitude of change in the thickness of the back film material 6, it is determined (step S7) that the tension condition is not to be changed, but there is no limitation to this, and the tension condition may be changed. In such a case, the timing for changing the tension condition is later than the timing for changing the back-film-material supply condition.

### List of Reference Numerals

1 absorbent article, 2 absorbent body
3 top sheet material, 3a material coil
5 back sheet material
6 back film material, 6a material coil
7 back-nonwoven-fabric-sheet material, 7a material coil
8 base sheet
9 elastic string, 9a elastic-string coil
10 absorbent-article manufacturing device, 12 first application device, 13 second application device
14 third application device, 15 fourth application device
21 fiber-depositing drum device, 21a rotating drum, 23 belt conveyor
25 absorbent-body pressing device, 25a press roller
27 embossing device, 27a embossing roller
27b first embossing device, 27c second embossing device
29 end cutter
31 inkjet device, 33 inspection camera device
35 tension adjustment device, 36 first tension adjustment device
37 second tension adjustment device, 38 third tension adjustment device

## Claims

1. A method for manufacturing an absorbent article (1) comprising:
manufacturing a first-specification absorbent article while transporting a material along a transporting route that corresponds to the material; and
switching from manufacturing of the first-specification absorbent article to manufacturing of a second-specification absorbent article by changing a plurality of types of manufacturing conditions at different positions in the transporting route while the transporting of the material is maintained,
the plurality of types of manufacturing conditions including:
a first manufacturing condition that is changed at a first position in the transporting route; and
a second manufacturing condition that is changed at a second position that is downstream of the first position,
the switching including setting timings for changing the manufacturing conditions so that a timing for changing the second manufacturing condition is later than a timing for changing the first manufacturing condition, wherein
while the absorbent article (1) is manufactured,
a sheet-shaped material is fed and transported as the material, from a material coil on which the sheet-shaped material is wound,
at least one of a width, a thickness, and a basis weight of the sheet-shaped material is changed in the switching as a manufacturing condition, and
depending on a magnitude of change in the at least one of the width, the thickness, and the basis weight,
it is determined whether or not a tension condition of the sheet-shaped material is to be changed in the switching as a manufacturing condition.

2. The method for manufacturing an absorbent article according to claim 1, wherein
in the switching, the plurality of types of manufacturing conditions are changed in order from an upstream side in the transporting route.

3. The method for manufacturing an absorbent article according to claim 1 or 2, wherein
a thickness of an absorbent body is different between the first-specification absorbent article and the second-specification absorbent article, and
depending on a magnitude of the difference in thickness, it is determined whether or not a pressing condition for pressing the absorbent body is to be changed in the switching as a manufacturing condition.

4. The method for manufacturing an absorbent article according to any of claims 1 to 3, wherein
a thickness of an absorbent body is different between the first-specification absorbent article and the second-specification absorbent article, and
depending on a magnitude of the difference in thickness, it is determined whether or not an embossing condition for forming embossing in the absorbent body is to be changed in the switching as a manufacturing condition.

5. The method for manufacturing an absorbent article according to any of claims 1 to 4, wherein
a basis weight of an absorbent body is different between the first-specification absorbent article and the second-specification absorbent article, and
depending on a magnitude of the difference in basis weight, it is determined whether or not a pressing condition for pressing the absorbent body is to be changed in the switching as a manufacturing condition.

6. The method for manufacturing an absorbent article according to any of claims 1 to 5, wherein
a basis weight of an absorbent body is different between the first-specification absorbent article and the second-specification absorbent article, and
depending on a magnitude of the difference in basis weight, it is determined whether or not an embossing condition for forming embossing in the absorbent body is to be changed in the switching as a manufacturing condition.

7. The method for manufacturing an absorbent article according to any of claims 1 to 6, wherein
the absorbent article includes an elastic member,
a stress of the elastic member is different between the first-specification absorbent article and the second-specification absorbent article, and
depending on a magnitude of the difference in stress, it is determined whether or not an adhesive application condition for applying an adhesive to the elastic member is to be changed in the switching as a manufacturing condition.

8. The method for manufacturing an absorbent article according to any of claims 1 to 7, wherein
while the absorbent article (1) is manufactured, inspection of an absorbent body (2) is executed by an inspection unit,
the absorbent body (2) includes pulp fibers,
a basis weight of the pulp fibers is different between the first-specification absorbent article and the second-specification absorbent article, and
depending on a magnitude of the difference in basis weight of the pulp fibers, it is determined whether or not an inspection condition of the inspection unit is to be changed in the switching as a manufacturing condition.

## Patentansprüche

1. Verfahren zur Herstellung eines saugfähigen Artikels (1), umfassend:
Herstellen eines saugfähigen Artikels einer ersten Spezifikation, während ein Material entlang einer Transportroute transportiert wird, die dem Material entspricht; und
Umschalten vom Herstellen des saugfähigen Artikels der ersten Spezifikation auf Herstellen eines saugfähigen Artikels einer zweiten Spezifikation durch Ändern einer Vielzahl von Arten von Herstellungsbedingungen an unterschiedlichen Positionen auf der Transportroute, während das Transportieren des Materials aufrechterhalten wird,
wobei die Vielzahl von Arten von Herstellungsbedingungen Folgendes beinhaltet:
eine erste Herstellungsbedingung, die an einer ersten Position auf der Transportroute geändert wird; und
eine zweite Herstellungsbedingung, die an einer zweiten Position geändert wird, die der ersten Position nachgeschaltet ist,
wobei das Umschalten Festlegen von Zeitpunkten zum Ändern der Herstellungsbedingungen beinhaltet, sodass ein Zeitpunkt zum Ändern der zweiten Herstellungsbedingung später ist als ein Zeitpunkt zum Ändern der ersten Herstellungsbedingung, wobei
während der saugfähige Artikel (1) hergestellt wird,
ein bahnförmiges Material als das Material von einer Materialrolle, auf die das bahnförmige Material aufgewickelt ist, eingespeist und transportiert wird,
mindestens eines von einer Breite, einer Dicke und einem Flächengewicht des bahnförmigen Materials beim Umschalten als Herstellungsbedingung geändert wird und
in Abhängigkeit von einem Ausmaß der Änderung des mindestens einen von der Breite, der Dicke und dem Flächengewicht
bestimmt wird, ob eine Spannungsbedingung des bahnförmigen Materials beim Umschalten als Herstellungsbedingung zu ändern ist oder nicht.

2. Verfahren zur Herstellung eines saugfähigen Artikels nach Anspruch 1, wobei
beim Umschalten die Vielzahl von Arten von Herstellungsbedingungen in einer Reihenfolge von einer vorgeschalteten Seite auf der Transportroute geändert wird.

3. Verfahren zur Herstellung eines saugfähigen Artikels nach Anspruch 1 oder 2, wobei
eine Dicke eines saugfähigen Körpers zwischen dem saugfähigen Artikel der ersten Spezifikation und dem saugfähigen Artikel der zweiten Spezifikation unterschiedlich ist und
in Abhängigkeit von einem Ausmaß des Unterschieds bei der Dicke bestimmt wird, ob eine Pressbedingung zum Pressen des saugfähigen Körpers beim Umschalten als Herstellungsbedingung zu ändern ist oder nicht.

4. Verfahren zur Herstellung eines saugfähigen Artikels nach einem der Ansprüche 1 bis 3, wobei
eine Dicke eines saugfähigen Körpers zwischen dem saugfähigen Artikel der ersten Spezifikation und dem saugfähigen Artikel der zweiten Spezifikation unterschiedlich ist und
in Abhängigkeit von einem Ausmaß des Unterschieds bei der Dicke bestimmt wird, ob eine Prägebedingung zum Bilden von Prägung in dem saugfähigen Körper beim Umschalten als Herstellungsbedingung zu ändern ist oder nicht.

5. Verfahren zur Herstellung eines saugfähigen Artikels nach einem der Ansprüche 1 bis 4, wobei
ein Flächengewicht eines saugfähigen Körpers zwischen dem saugfähigen Artikel der ersten Spezifikation und dem saugfähigen Artikel der zweiten Spezifikation unterschiedlich ist und
in Abhängigkeit von einem Ausmaß des Unterschieds bei dem Flächengewicht bestimmt wird, ob eine Pressbedingung zum Pressen des saugfähigen Körpers beim Umschalten als Herstellungsbedingung zu ändern ist oder nicht.

6. Verfahren zur Herstellung eines saugfähigen Artikels nach einem der Ansprüche 1 bis 5, wobei
ein Flächengewicht eines saugfähigen Körpers zwischen dem saugfähigen Artikel der ersten Spezifikation und dem saugfähigen Artikel der zweiten Spezifikation unterschiedlich ist und
in Abhängigkeit von einem Ausmaß des Unterschieds bei dem Flächengewicht bestimmt wird, ob eine Prägebedingung zum Bilden von Prägung in dem saugfähigen Körper beim Umschalten als Herstellungsbedingung zu ändern ist oder nicht.

7. Verfahren zur Herstellung eines saugfähigen Artikels nach einem der Ansprüche 1 bis 6, wobei
der saugfähige Artikel ein elastisches Element beinhaltet,
eine Beanspruchung des elastischen Elements zwischen dem saugfähigen Artikel der ersten Spezifikation und dem saugfähigen Artikel der zweiten Spezifikation unterschiedlich ist und
in Abhängigkeit von einem Ausmaß des Unterschieds bei der Beanspruchung bestimmt wird, ob eine Klebstoffauftragsbedingung zum Auftragen eines Klebstoffs auf das elastische Element beim Umschalten als Herstellungsbedingung zu ändern ist oder nicht.

8. Verfahren zur Herstellung eines saugfähigen Artikels nach einem der Ansprüche 1 bis 7, wobei
während der saugfähige Artikel (1) hergestellt wird, eine Inspektion eines saugfähigen Körpers (2) durch eine Inspektionseinheit ausgeführt wird,
der saugfähige Körper (2) Zellstofffasern beinhaltet,
ein Flächengewicht der Zellstofffasern zwischen dem saugfähigen Artikel der ersten Spezifikation und dem saugfähigen Artikel der zweiten Spezifikation unterschiedlich ist und
in Abhängigkeit von einem Ausmaß des Unterschieds bei dem Flächengewicht der Zellstofffasern bestimmt wird, ob eine Inspektionsbedingung der Inspektionseinheit beim Umschalten als Herstellungsbedingung zu ändern ist oder nicht.

## Revendications

1. Procédé de fabrication d'un article absorbant (1) comprenant :
la fabrication d'un article absorbant de première spécification tout en transportant un matériau le long d'un itinéraire de transport qui correspond au matériau ; et
le passage de la fabrication de l'article absorbant de première spécification à la fabrication d'un article absorbant de seconde spécification en modifiant une pluralité de types de conditions de fabrication à différentes positions dans l'itinéraire de transport pendant que le transport du matériau est maintenu,
la pluralité de types de conditions de fabrication comportant :
une première condition de fabrication qui est modifiée au niveau d'une première position sur l'itinéraire de transport ; et
une seconde condition de fabrication qui est modifiée au niveau d'une seconde position qui est en aval de la première position,
le passage comportant le réglage de timings pour le changement des conditions de fabrication de telle sorte qu'un timing pour le changement de la seconde condition de fabrication soit postérieur à un timing pour changer la première condition de fabrication, dans lequel
pendant la fabrication de l'article absorbant (1),
un matériau en forme de feuille est alimenté et transporté en tant que matériau, à partir d'une bobine de matériau sur laquelle le matériau en forme de feuille est enroulé,
au moins l'un parmi une largeur, une épaisseur et un grammage du matériau en forme de feuille est modifié lors du passage en tant que condition de fabrication, et
en fonction de l'ampleur du changement dans l'au moins une des valeurs suivantes : largeur, épaisseur et grammage,
il est déterminé si une condition de tension du matériau en forme de feuille doit être modifiée ou non lors du passage en tant que condition de fabrication.

2. Procédé de fabrication d'un article absorbant selon la revendication 1, dans lequel
lors du passage, la pluralité de types de conditions de fabrication sont modifiés dans l'ordre depuis un côté en amont de l'itinéraire de transport.

3. Procédé de fabrication d'un article absorbant selon la revendication 1 ou 2, dans lequel
l'épaisseur d'un corps absorbant est différente entre l'article absorbant de première spécification et l'article absorbant de seconde spécification, et
en fonction de l'ampleur de la différence d'épaisseur, il est déterminé si une condition de pressage pour presser le corps absorbant doit ou non être modifiée lors du passage en tant que condition de fabrication.

4. Procédé de fabrication d'un article absorbant selon l'une quelconque des revendications 1 à 3, dans lequel
une épaisseur d'un corps absorbant est différente entre l'article absorbant de première spécification et l'article absorbant de seconde spécification, et
en fonction de l'ampleur de la différence d'épaisseur, il est déterminé si une condition de gaufrage destinée à former un gaufrage dans le corps absorbant doit ou non être modifiée lors du passage en tant que condition de fabrication.

5. Procédé de fabrication d'un article absorbant selon l'une quelconque des revendications 1 à 4, dans lequel
le grammage d'un corps absorbant est différent entre l'article absorbant de première spécification et l'article absorbant de seconde spécification, et
en fonction de l'ampleur de la différence de grammage, il est déterminé si une condition de pressage pour presser le corps absorbant doit ou non être modifiée lors du passage en tant que condition de fabrication.

6. Procédé de fabrication d'un article absorbant selon l'une quelconque des revendications 1 à 5, dans lequel
le grammage d'un corps absorbant est différent entre l'article absorbant de première spécification et l'article absorbant de seconde spécification, et
en fonction de l'ampleur de la différence de grammage, il est déterminé si une condition de gaufrage pour former un gaufrage dans le corps absorbant doit ou non être modifiée lors du passage en tant que condition de fabrication.

7. Procédé de fabrication d'un article absorbant selon l'une quelconque des revendications 1 à 6, dans lequel
l'article absorbant comporte un élément élastique,
une contrainte de l'élément élastique est différente entre l'article absorbant de première spécification et l'article absorbant de seconde spécification, et
en fonction de l'ampleur de la différence de contrainte, il est déterminé si une condition d'application d'adhésif pour appliquer un adhésif sur l'élément élastique doit être modifiée ou non lors du passage en tant que condition de fabrication.

8. Procédé de fabrication d'un article absorbant selon l'une quelconque des revendications 1 à 7, dans lequel
pendant la fabrication de l'article absorbant (1), l'inspection d'un corps absorbant (2) est exécutée par une unité d'inspection,
le corps absorbant (2) comporte des fibres de pulpe,
le grammage des fibres de pulpe est différent entre l'article absorbant de première spécification et l'article absorbant de seconde spécification, et
en fonction de l'ampleur de la différence de grammage des fibres de pulpe, il est déterminé si une condition d'inspection de l'unité d'inspection doit ou non être modifiée lors du passage en tant que condition de fabrication.
